# FASCICULE DE BREVET EUROPEEN

(11) **EP 0 818 200 B1**
(45) Date de publication et mention de la délivrance du brevet: **10.04.2002**
(21) Numéro de dépôt: 97401606.5
(22) Date de dépôt: 04.07.1997
(51) Int. Cl.: A61K 31/715

(54) **Utilisation d'un polyholoside dans une composition destinée à favoriser la desquamation de la peau, et composition le comprenant**
Verwendung eines Polysaccharids in einer Zusammensetzung zur Förderung der Abschuppung der Haut und Zusammensetzung, die ein Polysaccharid enthält.
Use of polysaccharide to stimulate desquamation of the skin and compositions containing it

(30) Priorité: 10.07.1996 FR 9608616
(43) Date de publication de la demande: 14.01.1998
(73) Titulaire: L'OREAL, 75008 Paris (FR)
(72) Inventeur: Pineau, Nathalie, 86000 Poitiers (FR); Breton, Lionel, 78000 Versailles (FR)
(74) Mandataire: Tezier Herman, Béatrice

(56) Documents cités:
- DE-A- 4 221 753
- H. WAGNER ET AL.: "Immunstimulierend wirkende Polysaccharide (Heteroglykane) aus höheren Pflanzen." DRUG RESEARCH, vol. 35, no. 7, 1985, pages 1069-1075, XP000646167

## Description

L'invention concerne l'utilisation en tant que composé actif dans une composition cosmétique ou pour la préparation d'une composition pharmaceutique, en particulier dermatologique, d'une quantité efficace d'au moins un polyholoside, ce polyholoside ou la composition étant destiné à favoriser la desquamation de la peau et/ou stimuler le renouvellement épidermique et/ou lutter contre le vieillissement de la peau. Elle se rapporte aussi à des compositions pour favoriser la desquamation de la peau et/ou stimuler le renouvellement épidermique et donc lutter contre le vieillissement cutané intrinsèque et/ou extrinsèque, ainsi qu'à un procédé de traitement non thérapeutique de la peau destiné à favoriser la desquamation et/ou lutter contre le vieillissement de la peau.

La desquamation est un phénomène naturel lié au fait que l'épiderme, qui constitue la couche supérieure de la peau, est en constante régénération. L'épiderme est constitué de plusieurs assises de cellules, dont la plus profonde est l'assise basale constituée de cellules indifférenciées. Au cours du temps, ces cellules vont différencier et migrer vers la surface de l'épiderme en constituant les différentes assises de celui-ci, jusqu'à former à la surface de l'épiderme les cornéocytes qui sont des cellules mortes qui s'éliminent par desquamation. Cette perte en surface est compensée par la migration de cellules de l'assise basale vers la surface de l'épiderme. Il s'agit du renouvellement perpétuel de la peau. Une élimination forcée de la couche cornée accélère le renouvellement et permet de lutter contre le vieillissement.
Dans le même temps ces cellules poursuivent leur différenciation dont le dernier stade est le cornéocyte. Il s'agit en fait de cellules mortes qui constituent la dernière couche de l'épiderme, c'est à dire la couche la plus externe encore appelée *stratum corneum.*

Le vieillissement cutané résultant d'effets sur la peau de facteurs intrinsèques ou extrinsèques, se traduit par l'apparition de rides et ridules, par le jaunissement de la peau qui développe un aspect parcheminé accompagné de l'apparition de taches pigmentaires, par la désorganisation des fibres d'élastine et de collagène entraînant une perte d'élasticité, de souplesse et de fermeté et par l'apparition de télangiectasies.

Certains de ces signes du vieillissement sont plus particulièrement liés au vieillissement intrinsèque ou physiologique, c'est-à-dire au vieillissement « normal» lié à l'âge ou chronobiologique, alors que d'autres sont plus spécifiques du vieillissement extrinsèque, c'est-à-dire du vieillissement provoqué d'une manière générale par l'environnement ; il s'agit plus particulièrement du photo-vieillissement dû à l'exposition au soleil, à la lumière ou à tout autre rayonnement.

L'invention s'intéresse au vieillissement intrinsèque ou physiologique ainsi qu'au vieillissement extrinsèque.

Les changements de la peau dus au vieillissement intrinsèque sont la conséquence d'une sénescence génétiquement programmée où interviennent des facteurs endogènes. Ce vieillissement intrinsèque provoque notamment un ralentissement du renouvellement des cellules de la peau, ce qui se traduit essentiellement par l'apparition d'altérations cliniques telles que la réduction du tissu adipeux sous-cutané et l'apparition de fines rides ou ridules, et par des changements histopathologiques tels qu'une augmentation du nombre et de l'épaisseur des fibres élastiques, une perte de fibres verticales de la membrane du tissu élastique, et la présence de grands fibroblastes irréguliers dans les cellules de ce tissu élastique.

Au contraire, le vieillissement extrinsèque entraîne des altérations cliniques telles que des rides épaisses et la formation d'une peau molle et tannée, et des changements histopathologiques tels qu'une excessive accumulation de matière élastique dans le derme supérieur et une dégénérescence des fibres de collagène.

On connaît dans l'art antérieur divers agents destinés à lutter contre le vieillissement cutané.

Ainsi, le brevet US-A-4603146 décrit l'emploi d'acide rétinoïque et de ses dérivés dans des compositions cosmétiques, en vue de lutter contre le vieillissement cutané.
Par ailleurs, de nombreux brevets et publications (voir par exemple la demande EP-A-413528) ainsi que de nombreuses compositions cosmétiques du commerce enseignent l'emploi des α-hydroxyacides comme l'acide lactique, l'acide glycolique ou encore l'acide citrique pour traiter le vieillissement cutané.

On connaît enfin les bêta-hydroxy-acides et plus spécialement l'acide salicylique ainsi que ses dérivés pour leur propriétés desquamantes (voir les documents WO-A-93/10756 et US-A-4 767 750).

Tous ces composés ont une action contre le vieillissement de la peau en favorisant la desquamation, c'est-à-dire l'élimination des cellules « mortes » situées à la surface de la couche cornée de l'épiderme. Cette propriété "desquamante" est aussi appelée, souvent à tort, propriété kératolytique.

Mais les composés de l'art antérieur présentent également des effets secondaires, qui consistent en des picotements, des tiraillements, des échauffements et des rougeurs désagréables pour l'utilisateur.

On constate donc que subsiste le besoin d'agents antivieillissement ayant une action au moins aussi efficace que celle des composés de l'art antérieur, mais ne présentant pas leurs inconvénients.

L'invention a pour but de pallier les inconvénients des solutions de l'art antérieur et de proposer une composition favorisant la desquamation de la peau et/ou stimulant le renouvellement épidermique, dont l'utilisation n'entraînerait pas de picotements, de tiraillements, d'échauffements ou de rougeurs désagréables pour l'utilisateur.

La demanderesse a trouvé de manière inattendue que l'application d'une quantité efficace d'au moins un polyholoside permet de favoriser la desquamation de la peau et/ou stimuler le renouvellement épidermique donc de lutter contre le vieillissement.

L'invention a donc pour premier objet l'utilisation en tant que composé actif dans une composition cosmétique ou pour la préparation d'une composition pharmaceutique, en particulier dermatologique, d'une quantité efficace d'au moins un polyholoside, ce polyholoside ou la composition étant destiné à favoriser la desquamation de la peau et/ou stimuler le renouvellement épidermique et donc lutter contre le vieillissement cutané intrinsèque et/ou extrinsèque.

L'invention concerne également une composition contenant au moins un polyholoside pour favoriser la desquamation de la peau et/ou stimuler le renouvellement épidermique et donc lutter contre le vieillissement cutané intrinsèque et/ou extrinsèque.

Un autre objet de l'invention est un procédé de traitement non thérapeutique de la peau destiné à favoriser la desquamation de la peau et/ou stimuler le renouvellement épidermique et donc lutter contre le vieillissement cutané intrinsèque et/ou extrinsèque.

L'utilisation d'un polyholoside selon l'invention présente en outre d'autres avantages. Cela permet d'obtenir une composition pour favoriser la desquamation de la peau peu ou non irritante et peu collante, qui présente de surcroît un toucher doux et agréable.
Un autre avantage procuré par l'utilisation d'un tel polyholoside est d'améliorer la stabilisation de la composition finale, lorsqu'elle se présente sous forme d'émulsion, grâce aux propriétés auto-émulsionnantes du polyholoside employé, en particulier lorsqu'il s'agit d'un polyholoside comprend un motif fucose.
De plus, l'incorporation d'un polyholoside dans des compositions, notamment cosmétiques, permet l'obtention d'une composition gélifiée sans ajout supplémentaire d'agent gélifiant classiquement utilisé. Le gel obtenu est lisse et onctueux.

Les saccharides, de formule Cₙ(H₂O)ₙ, sont généralement divisés en deux catégories : les oses ou sucres simples, et les osides ou association de plusieurs molécules.
Parmi les osides, on peut distinguer (1) les holosides qui sont formés uniquement de sucres et (2) les hétérosides qui sont constitués par un ou plusieurs oses et une partie non glucidique.
De plus, parmi les polyholosides, on peut encore distinguer les polyholosides homogènes qui résultent de l'association d'un même ose, et les polyholosides hétérogènes qui résultent soit de l'association d'oses différents, soit de l'association d'oses ayant la même formule chimique brute mais de configuration géométrique différente (isomères D et L par exemple), considérés également dans la présente description comme des oses différents.
C'est cette dernière catégorie, constituée des polyholosides hétérogènes, qui est plus particulièrement concernée par la présente invention.

Préférentiellement, selon l'invention on utilise un polyholoside hétérogène en tant qu'agent destiné à favoriser la desquamation de la peau et/ou stimuler le renouvellement épidermique et donc lutter contre le vieillissement cutané intrinsèque et/ou extrinsèque.

Le polyholoside hétérogène selon l'invention est constitué uniquement de sucres et résulte de l'association d'au moins deux oses différents.
Les polyholosides selon l'invention peuvent être constitués de 2 à 10 oses, composés couramment appelés oligoholosides, ou de plus de 10 oses, composés couramment appelés polyholosides.
Les oses présents dans le polyholoside selon l'invention peuvent être choisis parmi tous les oses envisageables, d'origine naturelle ou synthétique, et notamment tels que :
. les aldoses comme
   . les pentoses : ribose, arabinose, xylose ou apiose, par exemple,
   . les hexoses : glucose, fucose, mannose ou galactose, par exemple,
. les cétoses tels que le fructose,
. les désoxyoses, tels que le rhamnose, le digitoxose, le cymarose ou l'oléandrose,
. les dérivés d'oses tels que les acides uroniques comme les acides mannuronique, guluronique, galacturonique ou glycuronique, ou encore les itols comme le mannitol ou le sorbitol.

On utilise de préférence selon l'invention un polyholoside hétérogène pouvant comprendre au moins deux oses choisis parmi le ribose, l'arabinose, le xylose, l'apiose, le glucose, le fucose, le mannose, le galactose, le fructose, le digitoxose, le cymarose, l'oléandrose, les acides uroniques et les itols.

Préférentiellement, le polyholoside selon l'invention peut comprendre au moins un motif fucose.

Encore plus préférentiellement le polyholoside comprend des motifs fucose, galactose et acide galacturonique et plus particulièrement un enchaînement linéaire de α-L-Fucose, de α-D-Galactose et d'acide galacturonique.

De préférence, le polyholoside hétérogène comprend au moins un motif fucose, qui peut être présent en une quantité de 10-90% en poids, de préférence 15-35% en poids, par rapport au poids de matière sèche de polyholoside.

Dans le cadre de la présente invention, on peut utiliser un polyholoside hétérogène seul, ou un mélange de polyholosides hétérogènes.

Le polyholoside selon l'invention peut être un alginate (poly mannuronate et guluronate) tel qu'un alginate de sodium, un alginate de propylène glycol, un alginate de calcium, ou un alginate de glycéryle.

Le polyholoside selon l'invention peut être éventuellement ramifié ou linéaire. Il peut également être substitué, par exemple par des chaînes grasses, notamment comprenant 8 à 30 atomes de carbone.

Le polyholoside de l'invention peut être de toute origine, naturel ou synthétique. Particulièrement, on peut utiliser selon l'invention un polyholoside préparé à partir de micro-organisme, comme par exemple *Klebsiella pneumoniae.* Encore plus particulièrement on utilise selon l'invention un polyholoside préparé à partir de la souche *Klebsiella pneumoniae subsp. Pneumoniae* dite BEC1000.

Lorsque l'on utilise un polyholoside d'origine naturelle, produit à partir d'un micro-organisme, il est généralement associé à des protéines. Il peut alors être soumis à un traitement protéolytique.
Toute technique permettant l'hydrolyse des protéines peut alors être mise en oeuvre. On citera de préférence l'hydrolyse enzymatique.

Le polyholoside peut être présent dans la composition finale en une quantité de 10⁻³ à 25% en poids par rapport au poids de la composition et de préférence de 10⁻² à 15% en poids par rapport au poids de la composition.

Le polyholoside présente de préférence une viscosité de 800-1200 mPa.s (viscosité Brookfield LV31, 12 tours/min, à 30°C) lorsqu'il est mis en solution dans l'eau à une concentration d'environ 1% en poids.

Selon un autre aspect, l'invention à pour objet une composition pour favoriser la desquamation de la peau et/ou stimuler le renouvellement épidermique et donc lutter contre le vieillissement cutané intrinsèque et/ou extrinsèque, comprenant au moins un polyholoside tel que défini précédement, autre qu'un alginate.

Dans la composition selon l'invention le polyholoside peut être présent en une quantité de 10⁻³ à 25% en poids, de préférence 10⁻² à 15% en poids, par rapport au poids de la composition.

Les polyholosides selon l'invention peuvent donc être utilisés en tant qu'agent destiné à favoriser la desquamation de la peau et/ou stimuler le renouvellement épidermique et donc lutter contre le vieillissement cutané intrinsèque et/ou extrinsèque, en particulier dans une composition à usage capillaire ou dans une composition pour la peau du corps et/ou du visage.

Ladite composition peut se présenter sous la forme d'une émulsion, notamment huile-dans-eau ou eau-dans-huile, voire sous la forme d'une émulsion multiple.
Elle peut également se présenter sous forme d'une solution aqueuse, éventuellement gélifiée, ou sous forme d'une lotion, par exemple biphasée, d'une crème, d'un lait, voire d'une mousse.

La composition selon l'invention peut comprendre une phase huileuse à base d'huile animale, végétale, minérale, siliconée, fluorée et/ou synthétique.
La phase huileuse peut également comprendre des alcools gras ou des acides gras, ainsi que des tensioactifs.
On peut notamment citer les huiles hydrocarbonées telles que l'huile de paraffine ou de vaseline; le perhydrosqualène; l'huile d'arara, l'huilé d'amande douce, de calophyllum, de palme, de ricin, d'avocat, de jojoba, d'olive ou de germes de céréales; des alcools tels que l'alcool oléique, l'alcool linoléique ou linolénique, l'alcool isostéarique ou l'octyl dodécanol. On peut également citer les huiles siliconées telles que les PDMS, éventuellement phénylées telles que les phényltriméthicones.
La phase huileuse peut également comprendre une huile démaquillante telle qu'un ester d'acides gras, en particulier les esters obtenus à partir d'un alcool à chaîne droite ou ramifiée ayant de 1 à 17 atomes de carbone et d'un acide gras à chaîne droite ou ramifiée ayant de 3 à 18 atomes de carbone.
Un tel ester peut en particulier être choisi dans le groupe constitué par l'adipate de dioctyle, le palmitate d'éthyl-2 hexyle, l'adipate de diisopropyle, l'hexanoate d'éthyl-2 hexyle, le laurate d'éthyle, le myristate de méthyle, l'octanoate d'octyldodécyle, le néopentanoate d'isodécyle, le myristate d'éthyle, le propionate de myristyle, l'éthyl-2 hexanoate d'éthyl-2 hexyle, l'octanoate d'éthyl-2 hexyle, le caprate/caprylate d'éthyl-2 hexyle, le palmitate de méthyle, le myristate de butyle, le myristate d'isobutyle, le palmitate d'éthyle, le laurate d'isohexyle, le laurate d'hexyle, l'isostéarate d'isopropyle.
La phase huileuse peut être présente à raison de 5-95% en poids dans le cas d'une émulsion.

La composition selon l'invention peut comprendre, en outre,
. un agent permettant la mise en suspension de la phase grasse, par exemple un copolymère d'acrylates d'alkyle en C₁₀-C₃₀ et d'acide acrylique ou méthacrylique ou de leur ester (Pemulen TR1, Pemulen TR2, Carbopol 1342 de GOODRICH); ou un copolymère acrylamide/acide méthylpropane sulfonique (Sepigel de SEPPIC), et/ou
. un agent de mise en dispersion de la phase grasse, tel qu'un système émulsionnant ou vésiculaire à base de vésicules, éventuellement de taille nanométrique, constituées de lipides ioniques (liposomes) ou non ioniques, et en particulier les systèmes émulsionnants bien connus de l'homme du métier constitués de stéarate de glycéryl /PEG 100 stéarate (CTFA), d'alcool cétylique et d'alcool stéarylique.

La composition de l'invention peut comprendre, en outre, un agent pour modifier sa viscosité, et obtenir des textures plus ou moins gélifiées, tel que :
. les dérivés cellulosiques (carboxyméthylcellulose, hydroxyéthylcellulose, hydroxypropylméthylcellulose),
. les gommes naturelles telles que les gommes de xanthane, de guar, de caroube, les scléroglucanes, les dérivés de chitine ou de chitosane, les carraghénanes,
. les dérivés polycarboxyvinyliques du type Carbomer (vendues par GOODRICH sous les dénominations Carbopol, 940, 951, 980, ou par 3V-SIGMA sous la dénomination Synthalen K ou Synthalen L).

La composition selon l'invention peut également comprendre, de façon connue, des adjuvants couramment utilisés dans le domaine considéré, tels que les conservateurs, les antioxydants, les parfums, les charges telles que le kaolin ou l'amidon, voire les microsphères creuses, les pigments, les filtres UV, les séquestrants, les huiles essentielles, les matières colorantes, les actifs hydrophiles ou lipophiles tels que les hydratants, notamment la glycérine, le butylène glycol, les anti-inflammatoires comme l'allantoïne, le bisabolol, les anti-radicaux libres comme la vitamine E ou ses dérivés, les apaisants tels que l'eau de bleuet, l'extrait d'iris, les dépigmentants, les actifs biologiques tels que l'urée, les acides aminés, les vitamines et leurs dérivés, les protéines, l'acide salicylique et ses dérivés, les α-hydroxy-acides, l'acide pyrrolidone carboxylique et ses sels, les céramides.
Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition présente de préférence un pH qui respecte la peau, généralement compris entre 5 et 8, de préférence un pH de 5,5 à 7,5.

L'invention est illustrée plus en détail dans les exemples suivants.

Exemple 1 : On étudie, dans cet exemple, la capacité d'un polyholoside riche en fucose (O.F.) selon l'invention à favoriser la desquamation.
Ce test de screening *in vitro* d'un agent actif sur la desquamation est réalisé sur kératinocytes humains différenciés. Le principe du test repose sur le fait que la desquamation induit la libération de cornéocytes. Le pouvoir de desquamation du produit testé va être d'autant plus grand que le nombre de cornéocytes libéré sera important.

Le protocole du test a été le suivant : à partir de biopsies de peau humaine, les kératinocytes obtenus par séparation de l'épiderme sont dissociés par action enzymatique à la trypsine et mis en culture à la concentration de 2.10⁵ cellules/ml. La croissance et la différenciation des kératinocytes a été obtenue par culture durant 10 à 20 jours en milieu spécifique. Puis, après élimination du milieu de culture, l'activité du produit à tester est évalué. Pour ce faire, deux prélèvements à T0 et T60 ont été réalisés, c'est-à-dire avant l'ajout du produit et 60 minutes après cet ajout. Les prélèvements ainsi effectués ont été analysés au cytomètre de flux pour dénombrer la population de coméocytes. Le cytomètre de flux permet de distinguer les populations de cornéocytes et de kératinocytes par traitement à l'acridine orange spécifique de l'ADN des cellules. Cette coloration est spécifique des kératinocytes puisque les cornéocytes normaux ne possèdent pas de noyaux donc par d'ADN.

L'indice de détachement cellulaire est déterminé par la différence entre T60 et T0. La même mesure a été réalisée pour un témoin ne contenant pas de produit à tester car l'expérience produit inévitablement la libération de cornéocytes, même en absence d'actifs.

Le test a été effectué sur un polyholoside préparé à partir de la souche *Klebsiella pneumoniae subsp. Pneumoniae,* et ayant subi un traitement protéolytique (O.F.). Cet polyholoside d'une masse molaire inférieure à 10.000Da est obtenu en laboratoire, après hydrolyse d'une fraction de polysaccharride bactérien.

Les résultats de ces études sont résumés dans le tableau suivant :

| | Référence* 5 10⁻⁵ M 0,00132% | O.F. 0,00132% | O.F. 0,000132% | O.F. 0,0000132% |
|---|---|---|---|---|
| O.F. | 108,7+/-4,2 | 207,9+/-6,1 | 139,1+/-6,7 | 30,5+/-3,6 |

| | | | | |
|---|---|---|---|---|
| * Référence : Acide 2-hydroxy-5-octanoylbenzoïque connu comme favorisant la desquamation (Brevet FR-85-06953 de la demanderesse) | | | | |

Les résultats sont donnés en % d'activité par rapport au témoin constitué d'une culture identique en l'absence de composé.
L'activité de O.F. sur le détachement cellulaire est donc importante. Cette activité dose dépendante est supérieure (en équivalent %) à celle de la référence.

Exemple 2 : Exemples de formulations illustrant l'invention. Ces compositions ont été obtenues par simple mélange des différents composants.

### Composition 1: Lait pour le visage

- Huile de vaseline 7,0 g
- O.F.* 10,0 g
- Monostéarate de glycéryle, stéarate de polyéthylène glycol (100 OE) 3,0 g
- Polymère carboxyvinylique 0,4 g
- Alcool stéarylique 0,7 g
- Protéines de soja 3,0 g
- NaOH 0,4 g
- Conservateur qs
- Eau qsp 100 g

Cette composition se présente sous forme d'un lait pour le visage, ayant de bonnes propriétés cosmétiques, et étant doux et confortable à utiliser.
Le pH de la composition est d'environ 5,5.

### Composition 2 : Lotion

- O.F.* 5,0 g
- Palmitate d'éthyl-2 hexyle 10,0 g
- Cyclopentadiméthylsiloxane 20,0 g
- Butylène glycol 5,0 g
- Conservateur qs
- Eau qsp 100 g

Cette lotion, qui ne contient pas de tensioactif, favorise la desquamation de la peau.

### Composition 3 : Lait

- Palmitate d'octyle 35,0 g
- Glycérine 2,0 g
- O.F.* 15,0 g
- Polymère réticulé acrylates C10-C30/alkylacrylates 0,1 g
- Triéthanolamine 0,1 g
- Acides aminés de blé 1,0 g
- Conservateur qs
- Eau qsp 100 g

Le lait obtenu, qui ne contient pas de tensioactif, possède de bonnes propriétés cosmétiques.

### Composition 4 : Gel pour le visage

- Glycérine 10,0 g
- O.F.* 20,0 g
- Cocoamphodiacétate de disodium 1,0 g
- Conservateur qs
- Eau qsp 100 g

Le gel obtenu possède de bonnes propriétés cosmétiques.

### Composition 5 : Gel nettoyant à l'eau

- Butylène glycol 7,0 g
- Sarcosinate de lauroyl sodium 4,0 g
- O.F.* 4,0 g
- Triéthanolamine 0,8 g
- Carbomer 0,5 g
- Conservateur qs
- Eau qsp 100 g

Le gel obtenu possède de bonnes propriétés cosmétiques.

O.F.* : polyholoside comprenant du fucose, du galactose et de l'acide galacturonique préparé à partir de la souche *Klebsiella pneumoniae subsp*. *Pneumoniae,* et ayant subi un traitement protéolytique, vendu par la société Solabia.

## Revendications

1. Utilisation en tant que composé actif dans une composition cosmétique ou pour la préparation d'une composition pharmaceutique, d'une quantité efficace d'au moins un polyholoside, ce polyholoside ou la composition étant destiné à favoriser la desquamation de la peau et/ou stimuler le renouvellement épidermique et donc lutter contre le vieillissement cutané intrinsèque et/ou extrinsèque.

2. Utilisation selon la revendication 1, **caractérisée en ce que** la composition pharmaceutique est une composition à usage dermatologique,

3. Utilisation selon l'une des revendications 1 ou 2, **caractérisée en ce que** le polyholoside est un polyholoside hétérogène.

4. Utilisation selon l'une quelconque des revendications précédentes, **caractérisée en ce que** le polyholoside hétérogène comprend au moins deux oses choisis parmi les aldoses, les cétoses, les désoxyoses, les dérivés d'oses et leurs mélanges.

5. Utilisation selon l'une quelconque des revendications précédentes, **caractérisée en ce que** le polyholoside hétérogène comprend au moins deux oses choisis parmi le ribose, l'arabinose, le xylose, l'apiose, le glucose, le fucose, le mannose, le galactose, le fructose, le digitoxose, le cymarose, l'oléandrose, les acides uroniques et les itols.

6. Utilisation selon l'une quelconque des revendications précédentes, **caractérisée en ce que** le polyholoside comprend au moins un motif fucose.

7. Utilisation selon l'une quelconque des revendications précédentes, **caractérisée en ce que** le polyholoside comprend des motifs fucose, galactose et acide galacturonique.

8. Utilisation selon l'une quelconque des revendications précédentes, **caractérisée en ce que** le polyholoside comprend un enchaînement linéaire de α-L-Fucose, de α-D-Galactose et d'acide galacturonique.

9. Utilisation selon l'une quelconque des revendications précédentes, **caractérisée en ce que** le polyholoside comprend un motif fucose présent en une quantité de 10-90% en poids, de préférence 15-35% en poids, par rapport au poids de matière sèche de polyholoside.

10. Utilisation selon l'une quelconque des revendications 1 à 5, **caractérisée en ce que** le polyholoside hétérogène est un alginate.

11. Utilisation selon la revendications précédente, **caractérisée en ce que** le polyholoside hétérogène est choisi parmi l'alginate de sodium, l'alginate de propylène glycol, l'alginate de calcium, ou l'alginate de glycéryle.

12. Utilisation selon l'une quelconque des revendications précédentes, **caractérisée en ce que** le polyholoside hétérogène est substitué par des chaînes grasses, ayant de préférence 8-30 atomes de carbone.

13. Utilisation selon l'une quelconque des revendications précédentes, **caractérisée en ce que** le polyholoside est préparé à partir de micro-organismes.

14. Utilisation selon la revendication précédente, **caractérisée en ce que** le polyholoside est préparé à partir *Klebsiella pneumoniae.*

15. Utilisation selon la revendication précédente, **caractérisée en ce que** le polyholoside est préparé à partir de la souche *Klebsiella pneumoniae subsp. Pneumoniae* (dite BEC1000).

16. Utilisation selon l'une quelconque des revendications précédentes, **caractérisée en ce que** le polyholoside à préalablement été soumis un traitement protéolytique.

17. Utilisation selon l'une quelconque des revendications précédentes, **caractérisée en ce que** le polyholoside est présent dans la composition finale en une quantité de 10⁻³ à 25% en poids,par rapport à la composition.

18. Utilisation selon l'une quelconque des revendications précédentes **caractérisée en ce que** le polyholoside est présent dans la composition finale en une quantité de 10⁻² à 15% en poids par rapport à la composition.

19. Utilisation selon l'une quelconque des revendications précédentes, dans une composition se présentant sous la forme d'une émulsion, d'une solution aqueuse, éventuellement gélifiée, d'une lotion notamment biphasée, d'une crème, d'un lait, d'une mousse.

20. Composition pour favoriser la desquamation de la peau et/ou stimuler le renouvellement épidermique et donc lutter contre le vieillissement cutané intrinsèque et/ou extrinsèque, **caractérisée en ce qu'**elle comprend au moins un polyholoside tel que défini dans l'une des revendications 3 à 16.

21. Composition selon la revendication 20, **caractérisée en ce que** le polyholoside est présent dans la composition finale en une quantité de 10⁻³ à 25% en poids, de préférence 10⁻² à 15% en poids, par rapport à la composition.

22. Procédé non thérapeutique pour favoriser la desquamation de la peau et/ou stimuler le renouvellement épidermique et donc lutter contre le vieillissement cutané intrinsèque et/ou extrinsèque, **caractérisé en ce qu'**on applique sur la peau une composition cosmétique telle que définie dans les revendications 20 ou 21.

## Claims

1. Use, as an active compound in a cosmetic composition or for the preparation of a pharmaceutical composition, of an effective amount of at least one polyholoside, this polyholoside or the composition being intended to promote desquamation of the skin and/or to stimulate epidermal renewal and thus to combat intrinsic and/or extrinsic ageing of the skin.

2. Use according to Claim 1, **characterized in that** the pharmaceutical composition is a composition for dermatological use.

3. Use according to either of Claims 1 and 2, **characterized in that** the polyholoside is a heterogeneous polyholoside.

4. Use according to any one of the preceding claims, **characterized in that** the heterogeneous polyholoside comprises at least two oses chosen from aldoses, ketoses, deoxyoses, ose derivatives and mixtures thereof.

5. Use according to any one of the preceding claims, **characterized in that** the heterogeneous polyholoside comprises at least two oses chosen from ribose, arabinose, xylose, apiose, glucose, fucose, mannose, galactose, fructose, digitoxose, cymarose, oleandrose, uronic acids and itols.

6. Use according to any one of the preceding claims, **characterized in that** the polyholoside comprises at least one fucose unit.

7. Use according to any one of the preceding claims, **characterized in that** the polyholoside comprises fucose, galactose and galacturonic acid units.

8. Use according to any one of the preceding claims, **characterized in that** the polyholoside comprises a linear chain of α-L-fucose, α-D-galactose and galacturonic acid.

9. Use according to any one of the preceding claims, **characterized in that** the polyholoside comprises a fucose unit present in an amount of 10-90% by weight, preferably 15-35% by weight, relative to the weight of polyholoside solids.

10. Use according to any one of Claims 1 to 5, **characterized in that** the heterogeneous polyholoside is an alginate.

11. Use according to the preceding claim, **characterized in that** the heterogeneous polyholoside is chosen from sodium alginate, propylene glycol alginate, calcium alginate and glyceryl alginate.

12. Use according to any one of the preceding claims, **characterized in that** the heterogeneous polyholoside is substituted with fatty chains preferably having 8-30 carbon atoms.

13. Use according to any one of the preceding claims, **characterized in that** the polyholoside is prepared from microorganisms.

14. Use according to the preceding claim, **characterized in that** the polyholoside is prepared from *Klebsiella pneumoniae.*

15. Use according to the preceding claim, **characterized in that** the polyholoside is prepared from the strain *Klebsiella pneumoniae subsp. Pneumoniae* (known as BEC1000).

16. Use according to any one of the preceding claims, **characterized in that** the polyholoside has been subjected to a prior proteolytic treatment.

17. Use according to any one of the preceding claims; **characterized in that** the polyholoside is present in the final composition in an amount of 10⁻³ to 25% by weight relative to the composition.

18. Use according to any one of the preceding claims, **characterized in that** the polyholoside is present in the final composition in an amount of 10⁻² to 15% by weight relative to the composition.

19. Use according to any one of the preceding claims, in a composition in the form of an emulsion, an optionally gelled aqueous solution, a lotion, in particular a two-phased lotion, a cream, a milk or a foam.

20. Composition for promoting desquamation of the skin and/or stimulating epidermal renewal and thus combating intrinsic and/or extrinsic ageing of the skin, **characterized in that** it comprises at least one polyholoside as defined in one of Claims 3 to 16.

21. Composition according to Claim 20, **characterized in that** the polyholoside is present in the final composition in an amount of 10⁻³ to 25% by weight, preferably 10⁻² to 15% by weight, relative to the composition.

22. Nontherapeutic process for promoting desquamation of the skin and/or stimulating epidermal renewal and thus combating intrinsic and/or extrinsic ageing of the skin, **characterized in that** a cosmetic composition as defined in Claim 20 or Claim 21 is applied to the skin.

## Patentansprüche

1. Verwendung einer wirksamen Menge mindestens eines Polyholosids als Wirkstoff in einer kosmetischen Zusammensetzung oder zur Herstellung einer pharmazeutischen Zusammensetzung, wobei das Polyholosid oder die Zusammensetzung zur Förderung der Abschuppung der Haut und/oder Stimulierung der Epidermiserneuerung und somit zur Bekämpfung der altersbedingten und/oder durch äußere Einwirkungen ausgelösten Hautalterung vorgesehen sind.

2. Verwendung nach Anspruch 1, **dadurch gekennzeichnet, daß** die pharmazeutische Zusammensetzung eine Zusammensetzung zur dermatologischen Verwendung ist.

3. Verwendung nach einem der Ansprüche 1 oder 2, **dadurch gekennzeichnet, daß** das Polyholosid ein heterogenes Polyholosid ist.

4. Verwendung nach einem der vorhergehenden Ansprüche, **dadurch gekennzeichnet, daß** das heterogene Polyholosid mindestens zwei Zucker enthält, die unter den Aldosen, Ketosen, Desoxyzuckern, Zuckerderivaten und deren Gemischen ausgewählt sind.

5. Verwendung nach einem der vorhergehenden Ansprüche, **dadurch gekennzeichnet, daß** das heterogene Polyholosid mindestens zwei Zucker enthält, die unter Ribose, Arabinose, Xylose, Apiose, Glucose, Fucose, Mannose, Galactose, Fructose, Digitoxose, Cymarose, Oleandrose, Uronsäuren und Zuckeralkoholen ausgewählt sind.

6. Verwendung nach einem der vorhergehenden Ansprüche, **dadurch gekennzeichnet, daß** das heterogene Polyholosid mindestens eine Fucoseeinheit enthält.

7. Verwendung nach einem der vorhergehenden Ansprüche, **dadurch gekennzeichnet, daß** das Polyholosid Fucose-, α-D-Galactose- und Galacturonsäureeinheiten enthält.

8. Verwendung nach einem der vorhergehenden Ansprüche, **dadurch gekennzeichnet, daß** das Polyholosid eine gerade Kette von α-L-Fucose, α-D-Galactose und Galacturonsäure enthält.

9. Verwendung nach einem der vorhergehenden Ansprüche, wobei das Polyholosid die Fucoseeinheit in einer Menge von 10 bis 90 Gew.-% und vorzugsweise 15 bis 35 Gew.-%, bezogen auf die Trockensubstanz des Polyholosids, enthält.

10. Verwendung nach einem der Ansprüche 1 bis 5, **dadurch gekennzeichnet, daß** das heterogene Polyholosid ein Alginat ist.

11. Verwendung nach dem vorhergehenden Anspruch, **dadurch gekennzeichnet, daß** das heterogene Polyholosid unter Natriumalginat, Propylenglykolalginat, Calciumalginat oder Glycerylalginat ausgewählt ist.

12. Verwendung nach einem der vorhergehenden Ansprüche, **dadurch gekennzeichnet, daß** das heterogene Polyholosid mit Fettketten substituiert ist, die vorzugsweise 8 bis 30 Kohlenstoffatome aufweisen.

13. Verwendung nach einem der vorhergehenden Ansprüche, **dadurch gekennzeichnet, daß** das Polyholosid aus Mikroorganismen erhalten wird.

14. Verwendung nach dem vorhergehenden Anspruch, **dadurch gekennzeichnet, daß** das Polyholosid aus *Klebsiella pneumoniae* erhalten wird.

15. Verwendung nach dem vorhergehenden Anspruch, **dadurch gekennzeichnet, daß** das Polyholosid aus *Klebsiella pneumoniae subsp. Pneumoniae* (BEC1000) erhalten wird.

16. Verwendung nach einem der vorhergehenden Ansprüche, **dadurch gekennzeichnet, daß** das Polyholosid vorab proteolytisch behandelt wird.

17. Verwendung nach einem der vorhergehenden Ansprüche, **dadurch gekennzeichnet, daß** das Polyholosid in der fertigen Zusammensetzung in einer Menge von 10⁻³ bis 25 Gew.-%, bezogen auf das Gesamtgewicht der Zusammensetzung, vorliegt.

18. Verwendung nach einem der vorhergehenden Ansprüche, **dadurch gekennzeichnet, daß** das Polyholosid in der fertigen Zusammensetzung in einer Menge von 10⁻² bis 15 Gew.-%, bezogen auf das Gesamtgewicht der Zusammensetzung, vorliegt.

19. Verwendung nach einem der vorhergehenden Ansprüche in einer Zusammensetzung, die in Form einer Emulsion, einer wäßrigen Lösung, die gegebenenfalls in ein Gel übergeführt ist, einer Lotion, insbesondere zweiphasigen Lotion, einer Creme, einer Milch oder eines Schaums vorliegt.

20. Zusammensetzung zur Förderung der Abschuppung der Haut und/oder Stimulierung der Epidermiserneuerung und daher zur Bekämpfung der altersbedingten und/oder durch äußere Einwirkungen ausgelösten Hautalterung, **dadurch gekennzeichnet, daß** sie mindestens ein Polyholosid nach einem der Ansprüche 3 bis 16 enthält.

21. Zusammensetzung nach Anspruch 20, **dadurch gekennzeichnet, daß** das Polyholosid in der fertigen Zusammensetzung in einer Menge von 10⁻³ bis 25 Gew.-%, bezogen auf die Zusammensetzung, und vorzugsweise 10⁻² bis 15 Gew.-% vorliegt.

22. Nicht therapeutisches Verfahren zur Förderung der Abschuppung der Haut und/oder Stimulierung der Epidermiserneuerung und daher zur Bekämpfung der altersbedingten und/oder durch äußere Einwirkungen ausgelösten Hautalterung, **dadurch gekennzeichnet, daß** auf die Haut einen kosmetische Zusammensetzung nach einem der Ansprüche 20 oder 21 aufgetragen wird.
